# EUROPEAN PATENT APPLICATION

(11) **EP 2 851 027 A1**
(43) Date of publication of application: **25.03.2015**
(21) Application number: 14160074.2
(22) Date of filing: 14.03.2014
(51) Int. Cl.: A61B 18/14, A61B 18/00

(54) **Ablation catheter devices and methods**

(30) Priority: 15.03.2013 US 201361793024 P
(71) Applicant: Tidal Wave Technology, Inc., Memphis, TN 38120 (US)
(72) Inventor: Himmelstein, Steven, Memphis, Tennessee 38120 (US); Sherman, Michael, Memphis, Tennessee 38120 (US); Stern, Roger, Cupertino, California 95014 (US); Jackson, Jerome, Los Altos, California 94024 (US)
(74) Representative: Prüfer & Partner GbR European Patent Attorneys

(57) **Abstract**

An ablation device for denervation including a catheter delivery mechanism including an elongated tube with a distal end and a proximal end, the distal end being emplaceable within a body lumen at a target nerve region. A guide wire, at least one radiofrequency electrode, a plurality of positioning elements, and a plurality of pressing elements initially located within the tube. The electrode being deployable from the tube at the target nerve region and forming a ringshaped structure adjacent the distal tube end. The positioning elements being deployable from the tube at the target nerve region from a position of the tube further distal than the electrode. The pressing elements being deployable from the tube more proximal than the electrode for use in pressing the deployed electrode against tissue to be ablated.

## Description

### FIELD

The present disclosure generally relates to a medical apparatus and method for treating neurovascular tissues through application of radiofrequency energy, and more particularly to an ablation apparatus for treating tissues in a patient.

### BACKGROUND

Arteries are the tube-shaped blood vessels that carry blood away from the heart to the body's tissues and organs and are each made up of outer fibrous layer, smooth muscle layer, connecting tissue and the inner lining cells (endothelium). Certain arteries comprise complex structures that perform multiple functions. For example, the aorta is a complex structure that performs multiple functions. Arteries are often associated with a local network of nerves that are involved in many bodily functions including maintaining vascular tone throughout the entire body and each individual organ, sodium and water excretion or reabsorption, as in the kidney, and blood pressure control. The electrical activity to these nerves originates within the brain and the peripheral nervous system.

The kidneys have a dense afferent sensory and efferent sympathetic innervation and are thereby strategically positioned to be the origin as well as the target of sympathetic activation. Communication with integral structures in the central nervous system occurs via afferent sensory renal nerves. Renal afferent nerves project directly to a number of areas in the central nervous system, and indirectly to the anterior and posterior hypothalamus, contributing to arterial pressure regulation. Renal sensory afferent nerve activity directly influences sympathetic outflow to the kidneys and other highly innervated organs involved in cardiovascular control, such as the heart and peripheral blood vessels, by modulating posterior hypothalamic activity. These afferent and efferent nerves traverse via the aorta to their destination end-organ site.

Some studies suggest that conditions such as renal ischemia, hypoxia, and oxidative stress result in increased renal afferent activity. Stimulation of renal afferent nerves, which may be caused by metabolites, such as adenosine, that are formed during ischemia, uremic toxins, such as urea, or electrical impulses, increases reflex in sympathetic nerve activity and blood pressure.

An increase in renal sympathetic nerve activity increases renin secretion rate, decreases urinary sodium excretion by increasing renal tubular sodium reabsorption, and decreases renal blood flow and glomerular filtration rate. When nervous activity to the kidney is increased, sodium and water are reabsorbed, afferent and efferent arterioles constrict, renal function is reduced, and blood pressure rises.

Renin release may be inhibited with sympatholytic drugs, such as clonidine, moxonidine, and beta blockers. Angiotensin receptor blockers substantially improve blood pressure control and cardiovascular effects. However, these treatments have limited efficacy and adverse effects. In addition, many hypertensive patients present with resistant hypertension with uncontrolled blood pressure and end organ damage due to their hypertension.

Patients with renal failure and those undergoing hemodialysis treatment exhibit sustained activation of the sympathetic nervous system, which contributes to hypertension and increased cardiovascular morbidity and mortality. Signals arising in the failing kidneys seem to mediate sympathetic activation in chronic renal failure. Toxins circulating in the blood as a result of renal failure cause excitation of renal afferent nerves and may produce sustained activation of the sympathetic nervous system.

Abrogation of renal sensory afferent nerves and renal efferent nerves has been demonstrated to reduce both blood pressure and organ-specific damage caused by chronic sympathetic overactivity in various experimental models. Hence, functional denervation of the human kidney by targeting both efferent sympathetic nerves and afferent sensory nerves appears to be a valuable treatment strategy for hypertension and perhaps other clinical conditions characterized by increased overall nerve activity and particularly renal sympathetic nerve. Functional denervation in human beings may also reduce the potential of hypertension related end organ damage.

Destruction or reduction in size of cellular tissues in situ has been used in the treatment of many diseases and medical conditions, both alone and as an adjunct to surgical removal procedures. This procedure is often less traumatic than surgical procedures and may be the only alternative where other procedures are unsafe or ineffective. This method, known as ablative treatment (or therapy), applies appropriate heat (or energy) to the tissues and causes them to shrink and tighten. Ablative treatment devices have the advantage of using a destructive energy that is rapidly dissipated and reduced to a nondestructive level by conduction and convection forces of circulating fluids and other natural body processes.

In many medical procedures, it is important to be able to ablate the undesirable tissue in a controlled and focused way without affecting the surrounding desirable tissue. Over the years, a large number of minimally invasive methods have been developed to selectively destroy specific areas of undesirable tissues as an alternative to resection surgery. There are a variety of techniques with specific advantages and disadvantages, which are indicated and contraindicated for various applications.

In one technique, elevated temperature (heat) is used to ablate tissue. When temperatures exceed 60°C, cell proteins rapidly denature and coagulate, resulting in a lesion. The lesion can be used to resect and remove the tissue or to simply destroy the tissue, leaving the ablated tissue in place. Heat ablation can also be performed at multiple locations to provide a series of ablations, thereby causing the target tissue to die and necrose. Subsequent to heating, the necrotic tissue is absorbed by the body or excreted.

Electrical currents may be used to create the heat for ablation of the tissue. Radiofrequency ablation (RF) is a high temperature, minimally invasive technique in which an active electrode is introduced in the target area, and a high frequency alternating current of up to 500 kHz, for instance, is used to heat the tissue to coagulation. Radiofrequency (RF) ablation devices work by sending current through the tissue, creating increased intracellular temperatures and localized interstitial heat.

RF treatment exposes a patient to minimal side effects and risks, and is generally performed after first locating the tissue sites for treatment. RF energy, when coupled with a temperature control mechanism, can be supplied precisely to the apparatus-to-tissues contact site to obtain the desired temperature for treating a tissue. By heating the tissue with RF power applied through one or more electrodes from a controlled radio-frequency (RF) instrument, the tissue is ablated.

The general theory behind and practice of RF heat lesion has been known for decades, and a wide range of RF generators and electrodes for accomplishing such practice exist. RF therapeutic protocol has been proven to be highly effective when used by electrophysiologists for the treatment of tachycardia, by neurosurgeons for the treatment of Parkinson's disease, and by neurosurgeons and anesthetists for other RF procedures such as Gasserian ganglionectomy for trigeminal neuralgia and percutaneous cervical cordotomy for intractable pains, as well as raziotomy for painful facets in the spine.

More recently denervation of the kidney has been studied due to its well know, positive impact on hypertension (high blood pressure). It can be accomplished, for example, via the renal artery ostium of the aorta, namely the orifice of the branch off the aorta that opens into the renal artery. Ablation of nerve activity at the level of the renal artery ostium will not affect blood flow from the aorta into the renal artery, but can cause the desired effect of denervation of the kidney. This kind of treatment is still relatively new, including what may be the best or desired treatment areas, and how to deliver the RF energy to the target area, which may be the area circumferentially surrounding the renal artery ostium. While the use of a catheter to deploy energy may be known for renal denervation, providing optimal uniform treatment is always a goal.

### SUMMARY

In general, it is an object of the present disclosure to provide a method and an improved medical ablation device for effectively ablating a nerve function of a subject or patient.

In an embodiment, the ablation device includes a catheter delivery mechanism including an elongated tube with a distal end and a proximal end, the distal end being placed within a body lumen at a target neurovascular region. A guide wire is disposed within the elongated tube. At least one radiofrequency electrode is initially located within the tube. The electrode being deployable from the tube at the target neurovascular region, and when deployed the electrode forms a ring-shaped structure generally centered about the tube adjacent the distal tube end. A plurality of positioning elements are initially located within the tube. The positioning elements are deployable from the tube at the target neurovascular region from a position of the tube further distal than the electrode. Pressing elements, initially located within the tube, are also deployable from the tube more proximal than the electrode for use in pressing, or positioning, the deployed electrode against tissue to be ablated. In one form, the tissue directly in contact with the electrode is cooled, thereby enabling targeting of an ablation deeper in the tissue without ablating the tissue in direct contact with the electrode. This is a case where the nerves being targeted are actually wrapped around the outside of the aorta and the renal arteries.

In an embodiment, a method for performing ablation of a neurovascular structure at an artery ostium, as in denervation, includes providing a catheter delivery mechanism including an elongated tube with a distal end and a proximal end, the distal end being emplaceable within a body lumen at a target neurovascular region, and having a guide wire within the elongated tube. Inserting the catheter delivery mechanism with its distal end at a target neurovascular region using the guide wire. At least one radiofrequency electrode initially located within the tube is provided, the electrode when deployed forming a ring-shaped structure generally centered about the tube adjacent the distal tube end. A plurality of positioning elements initially located within the tube are provided, the positioning elements being deployable from the tube at the target neurovascular region from a position of the tube further distal than the electrode. The positioning elements are then deployed to optimally position the electrode. The electrode is deployed at the target neurovascular region. A plurality of pressing elements initially located within the tube are provided, the pressing elements being deployable from the tube more proximal than the electrode for use in pressing the deployed electrode against tissue to be ablated to bring the electrode in close contact with the tissue. The electrode is pressed against the target neurovascular region, with radiofrequency energy applied through the deployed electrode from the tube at the target nerve region in an amount to ablate the targeted nerve region.

In another embodiment, a method for performing ablation of a renal nerve at the renal artery ostium includes providing a catheter delivery mechanism including an elongated tube with a distal end and a proximal end, the distal end being emplaceable within the body lumen at the renal artery ostium, and having a guide wire within the elongated tube for positioning the catheter delivery mechanism. The catheter delivery mechanism is inserted with its distal end at the renal ostium. At least one radiofrequency electrode initially located within the tube is provided, the electrode when deployed forms a ring-shaped structure generally centered about the tube adjacent the distal tube end. A plurality of positioning elements initially located within the tube are provided, the positioning elements being deployable from the tube in the renal artery at the ostium from a position of the tube further distal than the electrode. The positioning elements are deployed to optimally center the electrode. The electrode is deployed and a plurality of pressing elements initially located within the tube are provided, the pressing elements being deployable from the tube more proximal than the electrode for use in pressing the deployed electrode against ostium tissue. In one aspect, it is not the ostium but the tissue deep behind the ostium that is targeted to ablate. The electrode is then pressed against the ostial tissue, and radiofrequency energy is applied through the deployed electrode from the tube in a pre-specified amount to ablate the neurovascular tissue wrapped around a backside of the aorta and the renal artery.

In addition to the above noted functions, the device comprises a mechanism for cooling the aortic wall and the ostium in order to limit potential damage to the endothelial surface of the aorta while ablative energy is effectively transmitted to the adventitial layer. This cooling mechanism is by means of coolant or chilled material circulated through a hollow tube of the electrode, thus providing protection to the aortic wall at the level of the energy delivery. By cooling the tissue directly near the electrode, a target region deeper in the tissue (for example, tissue deep behind the ostium) can be ablated without ablating the tissue in direct contact with the electrode. This allows the target nerve region, a region wrapped around the outside of the aorta and the renal arteries, to be ablated when the device is deployed within the aorta and renal arteries.

The present disclosure is also directed to a method for radio-frequency (RF) heat ablation of tissue through the use of one or more RF electrodes, which are deployed from the distal end of a catheter. In a first step of the invention, the catheter may be inserted into the body via a natural orifice, a stoma or a surgically created opening that is made for the purpose of inserting the catheter, and insertion of the catheter may be facilitated with the use of a guide wire or a generic support structure or visualization apparatus. The catheter is advanced through the body to the relevant location, such as in the aorta at the location of the ostium of the renal artery.

RF energy is applied to the RF electrodes in order to effect changes in the target tissue. Heat is generated by supplying a suitable energy source to the apparatus, which is comprised of at least one electrode that is in contact with the body tissues. Additionally, coolant - either stagnant or circulating - may be employed to cool the inner surface of the vessel wall. This coolant function may provide a form of protection or insulation to the inner vessel wall surface during RF energy activation and heat transfer.

In one embodiment, the ablation is performed for the ablation of aortic nerve activity that leads specifically to the kidney.

### BRIEF DESCRIPTION OF DRAWINGS

Embodiments of devices, systems, and methods are illustrated in the figures of the accompanying drawings which are meant to be exemplary and not limiting, in which like references are intended to refer to like or corresponding parts, and in which:
FIG. 1 illustrates a first embodiment of an ablation device of the present application;
FIG. 2 illustrates an embodiment of a delivery catheter of the present application;
FIG. 3 illustrates an embodiment of an electrode of an ablation device of the present application in a deployed position;
FIG. 4 illustrates an embodiment of positioning and pressing elements of an ablation device of the present application;
FIG. 5 illustrates an embodiment of an ablation device of the present application in a deployed position;
FIG. 6 illustrates another embodiment of an electrode of an ablation device of the present application; and
FIG. 7 illustrates a schematic of an embodiment of system including an ablation device of the present application.

### DETAILED DESCRIPTION OF EMBODIMENTS

Detailed embodiments of devices, systems, and methods are disclosed herein, however, it is to be understood that the disclosed embodiments are merely exemplary of the devices, systems, and methods, which may be embodied in various forms. Therefore, specific functional details disclosed herein are not to be interpreted as limiting, but merely as a basis for the claims and as a representative basis for teaching one skilled in the art to variously employ the present disclosure.

As used herein, "proximal" refers to a portion of an instrument closer to an operator, while "distal" refers to a portion of the instrument farther away from the operator.

The term "subject" or "patient" refers in an embodiment to a mammal including a human in need of therapy for, or susceptible to, a condition or its sequelae. The subject or patient may include dogs, cats, pigs, cows, sheep, goats, horses, rats, and mice and humans.

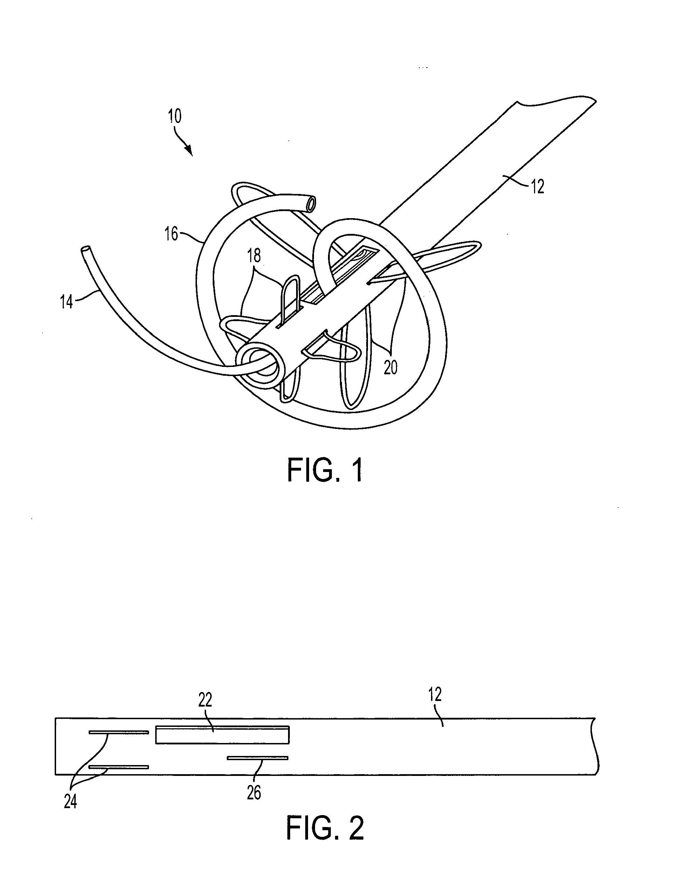

FIG. 1 illustrates an embodiment of an ablation device 10 for delivering radiofrequency energy to the walls of a body lumen. In an embodiment, the device is used for interaortic renal artery ablation for renal artery sympathetic neural ablation. Radiofrequency energy is delivered to the walls of the renal artery or aorta. In an embodiment of the device 10, radiofrequency energy is delivered using a nonconductive catheter.

In an embodiment, the device 10 includes a substantially tubular catheter 12, called a delivery catheter, namely an elongated, thin, tube-like device, having proximal and distal ends, preferably constructed from a nonconductive material. The delivery catheter 12 can be any type of catheter, as are well known to those in the art, having a proximal end for manipulation by an operator and a distal end for operation within a patient. The distal end and proximal end preferably form one continuous piece, but need not be in a single piece. In an embodiment, the delivery catheter 12 is used as a delivery system for delivering one or more radiofrequency electrodes to the desired site for nerve ablation. In an embodiment, the delivery catheter 12 has about a 2.55 mm outer diameter and about a 0.09 mm or less inner diameter.

The device 10 includes a guide wire 14 that may be advanced into the patient, e.g., through the delivery catheter 12. The guide wire 14, extends, through (within) the delivery catheter 12. The guide wire 14 here has a 0.035" thickness (or could employ other thicknesses as known in the art). The guide wire 14 is inserted into the patient's vascular system, e.g. through the groin, and advanced to the desired location. Next, the delivery catheter 12 is inserted into the patient and threaded over the guide wire 14 to the desired location. In one aspect, the device 10 may be advanced to the desired location within the patient's vascular system with, e.g., a rapid exchange (RX) or over-the-wire wire (OTW) delivery system, with the 0.035" or smaller guide wire 14 employed for the device 10. Radiographic control may be employed and contrast media may also be injected at the beginning of the procedure to assist in manipulation and positioning of the instruments.

In this embodiment, the device 10 includes one or more electrodes 16 deployable from the delivery catheter 12 adjacent the distal end of the delivery catheter 12. In this embodiment, a single electrode is used. The one or more electrodes 16 are capable of conducting radiofrequency (RF) energy. Initially, or when the one or more electrodes 16 are in a non-deployed position, the one or more electrodes 16 are located within the delivery catheter 12. The one or more electrodes 16, when deployed, from a ring-shape structure generally positioned in a circular configuration centered around the delivery catheter 12, such that the one or more electrodes 16 provide essentially 360° coverage at the target neurovascular region, for example, a renal artery ostium.

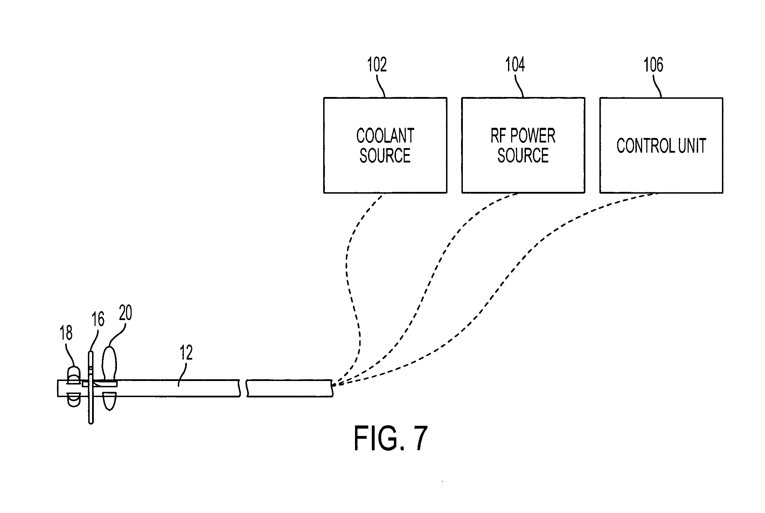

The electrode 16 is here in the form of a hollow tube, for example, a nitinol or other nickel-titanium alloy hypotube. The hollow tube 16 may be connected to a coolant source (e.g., coolant source 102 illustrated in FIG. 7), for example, a cold saline solution, and other coolants. The coolant may be circulated through the hollow tube, when performing the ablative function. The coolant may also be discharged into the patient through an end of the hypotube, and the coolant may be carried out of the patient through the patient's blood stream. The use of the coolant may assist in controlling the ablative temperature of or applied to the tissue to be ablated, and reduce thermal injury to the aorta and renal artery, in particular the intima of the vessels. By cooling the tissue directly near the electrode, a target region deeper in the tissue (for example, tissue deep behind the ostium) can be ablated without ablating the tissue in direct contact with the electrode. This allows the target nerve region, a region wrapped around the outside of the aorta and the renal arteries, to be ablated when the device is deployed within the aorta and renal arteries. Thus, the device can be used for interaortic renal artery ablation for renal artery sympathetic neural ablation.

The nickel-titanium alloy or nitinol hypotube of the electrode 16 is an alloy that has both super-elasticity and shape memory, i.e., remembers its original cold-forged shape, and returns to a pre-deformed shape when heated. This allows the electrode 16 to be deformed during the retracting and deploying of the electrode 16 into and from the delivery catheter 12, and when heated, for example, by application of radiofrequency (RF) energy, form the ring-shape structure described herein.

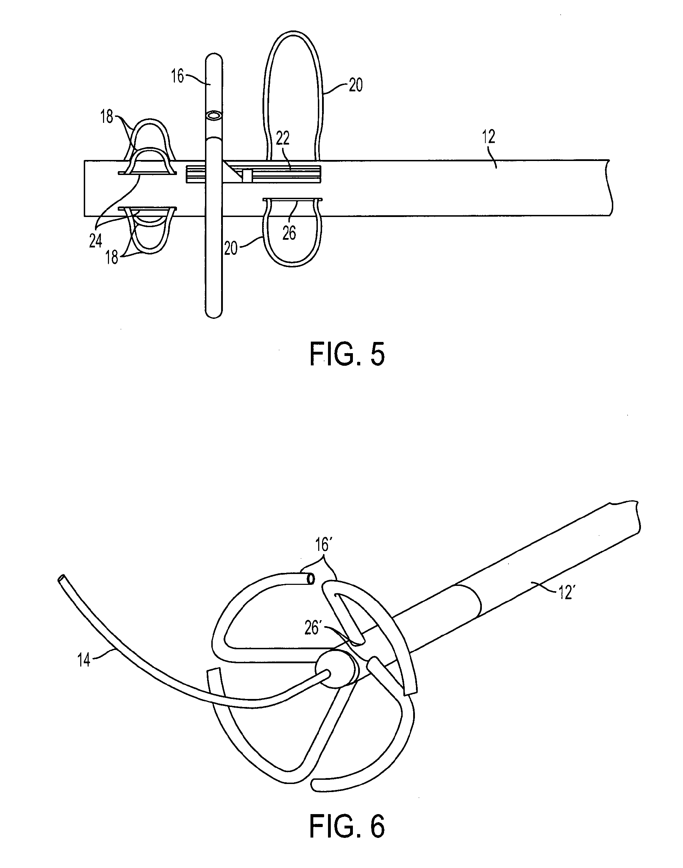

As illustrated in FIG. 1, there is one electrode 16. The electrode 16 includes a stem portion extending from an aperture (for example, an electrode aperture 22 described below), and a curved portion extending from the stem portion, forming ring-shape structure or arc around the delivery catheter 12. When more than one electrode 16 is used (for example, as described below with reference to FIG. 6), the electrodes 16 may be positioned such that, when the device 10 is in a deployed position, the electrodes 16 together form the ring-shape structure or are oriented concentrically, such that they together provide essentially 360° coverage around a target area. When electrode 16 is used, the more than one electrodes 16 may be nested or placed in parallel so as to form the ring-shape structure. Each of the nested electrodes 16 may include a stem portion, and a first portion curving or extending from the stem portion. The plural electrodes are spaced around the axis of the catheter so that the curved portions form a rough circular shape when deployed.

The one or more electrodes 16 may include a braid, coil, or laser cut tubular covering over the one or more electrodes 16. This tubular covering may be used in the deployment and retraction of the one or more electrodes 16 from the delivery catheter 12. The tubular covering may also function to adjust a diameter of the ring-shape structure to deploy the one or more electrodes 16 such that the ring-shape structure provides essentially 360° coverage around a target area.

The device 10 in this embodiment includes one or more positioning elements 18 deployable from the delivery catheter 12 adjacent the distal end of the delivery catheter 12. Initially, or when the one or more positioning elements 18 are in a non-deployed position, the one or more positioning elements 18 are located within the delivery catheter 12. The one or more positioning elements 18 are deployable from the delivery catheter 12 at a target region from a position of the delivery catheter 12 further distal than the one or more electrodes 16. In use, this allows the positioning elements 18 to position and secure device 10 at the desired location within a vessel, e.g., the aorta in the area of the renal artery ostium. The one or more positioning elements 18 are used so that the one or more electrodes 16 can operate at the precise location, namely around the renal artery ostium. Otherwise, if the device 10 is not properly positioned, the electrode(s) 16 could ablate tissue that is not intended to be affected, causing undesired damage. In an embodiment, where the RF electrode 16 is circularly configured, the positioning elements 18 should properly center the electrode 16 circumferentially around the renal artery ostium, namely the opening to the renal artery.

The one or more positioning elements 18 in this embodiment are wire loops and are located symmetrically around the delivery catheter 12. When the delivery catheter 12 is inserted at least partially into the entrance of the renal artery, the positioning elements 18 may be deployed approximately to the diameter of the renal artery, so as to locate the electrode 16 from being moved distally or proximally relative to the renal artery, so as to allow the device 10 to hold its position within the renal artery relative to the aorta. When the device 10 is so positioned by the positioning elements 18, the electrode 16 may then be positioned against the renal artery ostium to perform the ablative function, as will be shortly described.

The device 10 includes one or more pressing elements 20 deployable from the delivery catheter 12 proximal to the electrode 16. Initially, or when the one or more pressing elements 20 are in a non-deployed position, the one or more pressing elements 20 are located within the delivery catheter 12. The one or more pressing elements 20 are deployable from the delivery catheter 12 once the device is at a target nerve region, and from a position of the delivery catheter 12 more proximal than the one or more electrodes 16. When deployed, the pressing elements 20 may be used to press the deployed one or more electrodes 16 against the tissue to be ablated.

In one form, the pressing elements 20 are wire loops and are located symmetrically around the delivery catheter 12. The pressing elements 20 may advance the delivery catheter 12 distally such that the delivery catheter 12 presses against a proximally-facing surface of the one or more electrodes 16 to then be manipulated to push the one or more electrodes 16 distally against the renal artery ostium. When the one or more electrodes 16 are pressed up against the renal artery ostium of the aorta, the one or more electrodes 16, which are positioned in a circular configuration, contact the renal artery ostium of the aorta. Heat may then be generated to the one or more electrodes 16 by supplying a suitable RF energy source, and the ablation is performed for the elimination (or interruption) of nerve activity, such as nerve activity that leads specifically to the kidney.

Each of the one or more electrodes 16, the one or more positioning elements 18 and the one or more pressing elements 20 may be selectively and independently movable between a non-deployed position (or retracted) and a deployed position, and back to the non-deployed position. Alternatively, they could be joined in a manner such that they are deployed together as a group

(e.g., all of the positioning elements 18 are deployed together). In the non-deployed position, as illustrated in FIG. 2, the electrode 16, the positioning elements 18 and the pressing elements 20 of device 10 are retracted within the delivery catheter 12. As illustrated in FIG. 2, the delivery catheter 12 includes an electrode aperture 22, positioning element apertures 24 and pressing element apertures 26. The electrode aperture 22, the positioning element apertures 24 and the pressing element apertures 26 allow the electrode 16, the positioning elements 18 and the pressing elements 20 to be extended out of the delivery catheter 12, to their respective deployed positions.

In an embodiment, a distance between the distal end of the delivery catheter 12 and the electrode aperture 22 and/or the electrode 16 is about 10 mm to about 20 mm in length. A distance between the positioning element apertures 24 and the electrode aperture 22 and/or the electrode 16 is about 5 mm to about 7 mm in length.

In the non-deployed position, the delivery catheter 12 is advanced longitudinally through the blood vessel, e.g., over guide wire 14, to the relevant location within the body lumen, such as within the aorta, and into the desired position within the inner circumference of the vessel, such as at the renal artery ostium of the aorta. Once at the desired position, the electrode 16, the positioning elements 18 and the pressing elements 20 are deployed. Preferably, the positioning elements 18 are deployed first, then the electrode 16 followed by the pressing elements 20. This order need not be the only order, however.

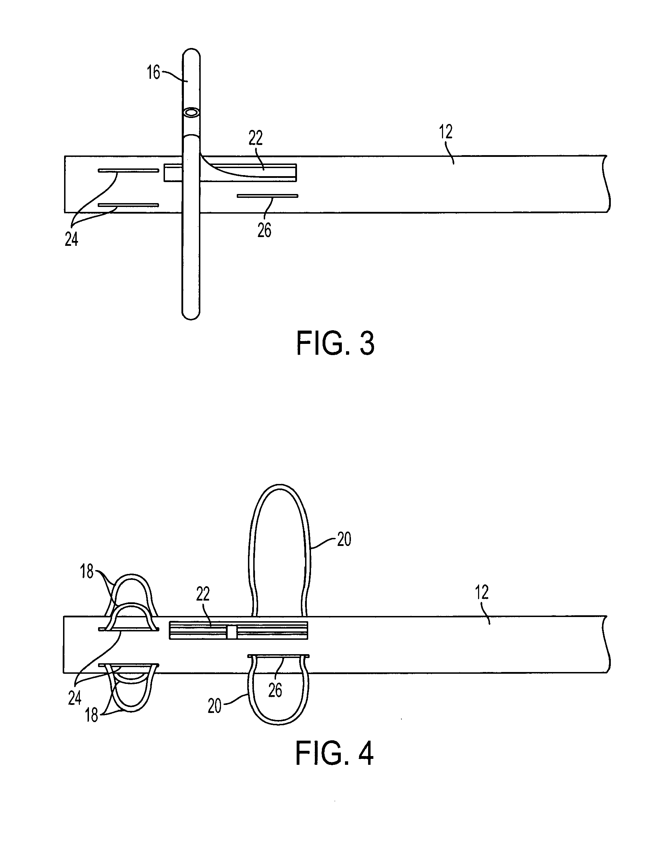

As illustrated in FIG. 3, the electrode 16 is in the deployed position for operation within the patient. In the deployed position, the electrode 16 extends out of the delivery catheter 12 through the electrode aperture 22, forming the ring-shape structure generally positioned in a circular configuration centered around the delivery catheter 12, such that the electrode 16 provides essentially 360° coverage at the target nerve region. The electrode 16 can be pressed up against and put into contact with the renal artery ostium of the aorta, for instance, to ablate the nerve activity circumferentially around the ostium.

As illustrated in FIGS. 4 and 5, the positioning elements 18 and the pressing elements 20 are in the deployed position, for operation within the patient. In the deployed position, the positioning elements 18 and the pressing elements 20 extend out of the delivery catheter 12 through the positioning element apertures 24 and the pressing element apertures 26, respectively. Referring to FIG. 5, the electrode 16, the positioning elements 18 and the pressing elements 20 are all in the deployed position for operation within the patient.

To return to the non-deployed position, as for withdrawal, the electrode 16, the positioning elements 18 and the pressing elements 20 are retracted into the inner diameter of delivery catheter 12.

In another embodiment, as illustrated in FIG. 6, the device includes more than one electrodes 16' deployable from a delivery catheter 12' adjacent the distal end of the delivery catheter 12'. The electrodes 16' are similarly capable of conducting RF energy. Initially, or when the electrodes 16' are in a non-deployed position, the electrodes 16' are located within the delivery catheter 12'. The electrodes 16', when deployed, are positioned such that, when the device is in a deployed position, the electrodes 16' together form a ring-shape structure, or are oriented concentrically, such that they together provide (perhaps roughly) essentially 360° coverage around a target area. As illustrated in FIG. 6, there are four electrodes 16', but there can be fewer electrodes or more electrodes, each of which include a stem portion extending radially from the respective aperture 26' in the delivery catheter 12', and a curved portion extending from the stem portion. The curved portions align to form a ring-shape structure or arc around the delivery catheter 12'. The electrodes 16' may also include the braid, coil, or laser cut tubular covering over the electrodes 16', as described above with reference to electrode 16.

The delivery catheter 12' also includes electrode apertures 26' to allow the electrodes 16' to be extended out of the delivery catheter 12' to their respective deployed positions. Although not shown, the device may also include the one or more positioning elements, the one or more pressing elements, and the delivery catheter 12' may include their respective apertures, such that the device functions is essentially the same manner as described above with respect to FIGS. 1-5.

In these embodiments, the positioning elements 18 operate to position, center, and secure the device at the desired location. This is accomplished by insertion of the unexpanded distal end of the delivery catheter 12/12' at least partially into the entrance of the renal artery so as to serve, by deployment of the positioning elements 18, as an anchor for the device within the aorta so that the electrodes 16/16' can perform their ablative function. Similarly, in these embodiments, the one or more pressing elements 20 operate to engage the one or more electrodes 16/16' at the desired location. This is accomplished by using the pressing elements 20 so as to push the one or more electrodes 16/16' against the tissue to be ablated so that the one or more electrodes 16/16' can perform their ablative function.

In an embodiment, the proximal end of the device includes at least one port for connection to a source of radiofrequency (RF) power (e.g., RF power source 104 illustrated in FIG. 7). The device can be coupled to a source of RF energy, such as RF in about the 300 kilohertz to 500 kilohertz range. The electrodes 16/16' may be electrically coupled to the RF energy source through this port. The device may also be connected to coolant source, and a control unit for sensing and measurement of other factors, such as temperature, conductivity, pressure, impedance and other variables, such as nerve energy.

In an embodiment, the one or more electrodes 16/16' are electrically connected to the radiofrequency (RF) energy source. The RF energy source may be an external RF control unit that provides RF energy to the one or more electrodes 16/16'. In an embodiment, all the electrodes 16/16' are attached to the same wire such that they are made to operate together. The electrodes 16/16' may also have wires that loosely connect them, in order for them to be connected electrically.

In another embodiment, there may be multiple wires, each of which is attached to one or more of the electrodes 16/16' so as to conduct RF energy from the RF control unit to individual electrodes 16/16'. This allows independent control of the electrodes 16/16' to deliver RF energy simultaneously or in a sequential or other desired pattern.

The one or more electrodes 16/16' operate to provide radiofrequency energy for heating of the desired location during the nerve ablation procedure. The one or more electrodes 16/16' may be constructed of any suitable conductive material, as is known in the art. Examples include stainless steel and platinum alloys.

As described above, the one or more electrodes 16/16' are in a preferred form, hollow tubes, for example, nitinol hypotubes. The nitinol hypotubes may be a 4 x 0.018 mm nitinol hypotube. The hollow tube may be connected to a coolant source (e.g., coolant source 102 illustrated in FIG. 7), for example, a cold saline solution, and other coolants both gas and liquid. The coolant is circulated through the hollow tube, when performing the ablative function. This may assist in controlling the ablative temperature applied to the tissue to be ablated, and reduce thermal injury to the aorta and renal artery. For example, this may limit the thermal effect to about a 3 mm to about a 6 mm depth, for example, from the level of the renal artery ostium.

The cooling allows a target region deeper in the tissue (for example, tissue deep behind the ostium) to be ablated without ablating the tissue in close proximity to the electrode. This allows the target nerve region, a region wrapped around the outside of the aorta and the renal arteries, to be ablated.

The one or more electrodes 16/16' may operate in either bipolar or monopolar mode, with a ground pad electrode. In a monopolar mode of delivering RF energy, a single electrode is used in combination with an electrode patch that is applied to the body to form the other electrical contact and complete an electrical circuit. A bipolar operation is possible when two or more electrodes are used, such as two concentric electrodes. The one or more electrodes 16/16' can be attached to an electrode delivery member, such as the wire frame, by the use of soldering or welding methods which are well known to those skilled in the art.

The one or more electrodes 16/16' are oriented in a generally circular configuration. The diameter of the circular or ring-shape of the electrodes 16/16' is determined by the width of the aortic artery branch for which denervation is desired. If the diameter of the circular or ring-shape of the electrodes 16/16' is smaller than the diameter of the aortic artery branch for which denervation is desired, the one or more electrodes 16/16' would not actually be in contact with tissue, and no ablation would occur. For example, when aortic denervation is desired at the level of the renal artery ostium, which is approximately 6-7 mm in diameter at the ostium of the aorta, the diameter of the circular or ring-shape of the electrodes 16/16' should be at least that distance, i.e., 7 mm, in order to properly provide ablation surrounding the renal artery ostium. The diameter of the circular or ring-shape of the electrodes 16/16' may be calculated with reference to the renal artery ostium. For example, if it is desired that the RF energy be applied at least approximately 2 mm from each edge of the renal artery ostium, the diameter of the circular or ring-shape of the electrodes 16/16' that surround the imaging catheter may have a 10 mm to about a 15 mm diameter.

The one or more electrodes 16/16' can be disposed to treat tissue by delivering radiofrequency (RF) energy. The radiofrequency energy delivered to the electrode may have a frequency of about 5 kilohertz (kHz) to about 1 GHz. In specific embodiments, the RF energy may have a frequency of about 10 kHz to about 1000 MHz; specifically about 10 kHz to about 10 MHz; more specifically about 50 kHz to about 1 MHz; even more specifically about 300 kHz to about 500 kHz.

In a preferred embodiment, each electrode can be operated separately or in combination with another as sequences of electrodes disposed in arrays. Treatment can be directed at a single area or several different areas of a vessel by operation of selective electrodes. An electrode selection and control switch may include an element that is disposed to select and activate individual electrodes.

The RF power source may have multiple channels, delivering separately modulated power to each electrode. This reduces preferential heating that occurs when more energy is delivered to a zone of greater conductivity and less heating occurs around electrodes that are placed into less conductive tissue. If the level of tissue hydration or the blood infusion rate in the tissue is uniform, a single channel RF power source may be used to provide power for generation of lesions relatively uniform in size.

The RF energy delivered through the electrodes to the tissue causes heating of the tissue due to absorption of the RF energy by the tissue and ohmic heating due to electrical resistance of the tissue. This heating can cause injury to the affected cells and can be substantial enough to cause cell death, a phenomenon also known as cell necrosis. For ease of discussion, "cell injury" includes all cellular effects resulting from the delivery of energy from the electrodes up to, and including, cell necrosis. Use of the catheter device can be accomplished as a relatively simple medical procedure with local anesthesia. In an embodiment, cell injury proceeds to a depth of approximately 1-5 mm from the surface of the mucosal layer of sphincter or that of an adjoining anatomical structure.

Also to be potentially included in this design is a means to measure renal nerve afferent activity prior to and following RF nerve ablation. By measuring renal nerve activity post procedure, a degree of certainty is provided that proper nerve ablation has been accomplished. Renal nerve activity may be measured through the same mechanism as that required for energy delivery and the electrodes.

Nerve activity may be typically measured by one of two means. Proximal nerve stimulation can occur by means of transmitting an electrical impulse to the catheter. Action potentials can be measured from the segment of the catheter situated within a more distal portion of the nerve. The quantity of downstream electrical activity as well as the time delay of electrical activity from the proximal to distal electrodes will be provide a measure of residual nerve activity post nerve ablation. The second means of measuring nerve activity is to measure ambient electrical impulses prior to and post nerve ablation within a site more distal than the ablation site.

In an embodiment, the one or more electrodes 16/16' operate to provide radiofrequency energy for both heating and temperature sensing. Thus, in this embodiment, the one or more electrodes 16/16' can be used for heating during the ablation procedure and can also be used for sensing of nerve activity prior to ablation as well as after ablation has been done.

The one or more electrodes 16/16' may also be coupled to a sensor or a control unit (e.g., control unit 106 illustrated in FIG. 7) capable of measuring such factors as temperature, conductivity, pressure, impedance and other variables. For example, the device may have a thermistor that measures temperature in the lumen, and a thermistor may be a component of a microprocessor-controlled system that receives temperature information from the thermistor and adjusts wattage, frequency, duration of energy delivery, or total energy delivered to the one or more electrodes 16/16'. In other words, a closed loop, feedback control system may be incorporated to optimize the delivery of ablative energy to the tissue.

The device may also be coupled to a visualization apparatus, such as a fiber optic device, a fluoroscopic device, an anoscope, a laparoscope, an endoscope or the like. In an embodiment, devices coupled to the visualization apparatus are controlled from a location outside the body, such as by an instrument in an operating room or an external device for manipulating the inserted catheter.

In another embodiment, the device may be constructed with markers that assist the operator in obtaining a desired placement, such as radio-opaque markers, etchings or microgrooves. Thus, device may be constructed to enhance its imageability by techniques such as ultrasounds, CAT scan or MRI. In addition, radiographic contrast material may be injected through a hollow interior of the catheter through an injection port, thereby enabling localization by fluoroscopy or angiography.

The disclosure herein also comprises a method for ablation of renal artery nerve function within the aorta using the devices described herein. In an embodiment, a method for performing ablation of a nerve at an artery ostium includes inserting a distal end of a device, for example, device 10 including the delivery catheter 12/12', at a target nerve region using a guide wire. The targeted neurovascular region may be the renal artery ostium.

This method includes deploying one or more positioning elements, for example, positioning elements 18, from the delivery catheter 12/12' to position the device and an electrode, for example, electrodes 16/16', for deployment within the target nerve region. As described above, the positioning elements may center and secure the device, for example, the delivery catheter 12/12', in the target nerve region.

The method includes deploying the electrode, for example, electrodes 16/16', from the delivery catheter 12/12' at the target nerve region. When deployed, the electrode may form a ring-shaped structure generally centered around the delivery catheter 12/12' adjacent the distal end. The ring-shaped structure may also extend substantially circumferentially around the target nerve region.

The method of this embodiment includes deploying one or more pressing elements, for example, pressing elements 20, from the delivery catheter 12/12' (either before or after electrode deployment) at a position more proximal than the electrode, for example, electrodes 16/16'. As described above, the pressing elements may be used for pressing the deployed electrode, for example, electrodes 16/16', against tissue to be ablated at the target nerve region. In an embodiment, the method may also include pressing the deployed electrode, for example, electrodes 16/16', against tissue at the target nerve region.

Radiofrequency (RF) energy is applied through the deployed electrode, for example, electrodes 16/16', in an amount to ablate tissue at the target nerve region. The radiofrequency energy may be applied at a single energy level for a defined and regulated period of time or at a first energy level and at least a second energy level which is different from the first energy level. The first and second energy levels may be alternated and pulsed. Further, there may be a defined pause between the delivery of each energy level to allow the tissue temperature to normalize.

The method may include circulating a coolant through the hollow tube electrodes during the ablation procedure.

In an embodiment, the method includes the step of precooling the target nerve area, for example by circulating the coolant through the hollow tube electrodes. The precooling may be performed for any period of time, particularly about 10 seconds to about 20 seconds, and more particularly for about 15 seconds. Following the precooling step, the radiofrequency energy may be applied at the first energy level. The first energy level is about 1.4 amps, and is applied for about 60 seconds to about 90 seconds. Following the application of the radiofrequency energy at the first energy level, the radiofrequency energy may be applied at the second energy level. The second energy level is about 1.2 amps, and is applied for about 90 seconds. A pause may also be incorporated between the delivery of the first and second energy level.

In an embodiment, the ablation procedure incudes applying the radiofrequency energy at a first energy level for a first period of time, followed by a rest and then applying the radiofrequency energy at a second energy level for a second period of time. The first energy level and the second energy level may be equal. Similarly, the first period of time and the second period of time may be equal.

Although the method steps are described herein serially, there is no particular requirement that the method be performed in the same order in which this description lists the steps, except where so indicated.

Although the devices, systems, and methods have been described and illustrated in connection with certain embodiments, many variations and modifications will be evident to those skilled in the art and may be made without departing from the spirit and scope of the disclosure. The discourse is thus not to be limited to the precise details of methodology or construction set forth above as such variations and modification are intended to be included within the scope of the disclosure.

## Claims

1. An ablation device, as for sympathetic aortic and renal artery denervation, comprising:
a catheter delivery mechanism including an elongated tube with a distal end and a proximal end, said distal end being emplaceable within an arterial system for delivery within an aorta at a level of a renal artery ostium; and
at least one radiofrequency electrode initially located within said tube, said electrode being deployable from said tube, said electrode when deployed forming a ring-shaped structure generally centered about said tube adjacent said distal tube end.

2. The ablation device of Claim 1, further including at least one positioning element initially located within said tube, said at least one positioning element being deployable from said tube from a position of said tube further distal than said electrode.

3. The ablation device of Claim 1, further including at least one pressing element initially located within said tube, said at least one pressing element being deployable from said tube more proximal than said electrode for use in pressing said deployed electrode against tissue to be ablated.

4. The ablation device of Claim 1 or 2, further including a source of radiofrequency energy connected to said electrode.

5. The ablation device of one of Claims 1 to 4, wherein said electrode is a hollow tube.

6. The ablation device of Claim 5, further including a source of coolant, wherein said coolant is circulated through said electrode tube.

7. The ablation device of one of Claims 1 to 6, wherein said electrode is comprised of a plurality of separate electrode members each of which is deployable from said tube, and together take a ring-like shape when deployed.

8. The ablation device of Claim 7, wherein said electrode members are in the form of hollow tubes, further including a source of coolant, wherein said coolant is circulated through said electrode tube members.

9. The ablation device of one of Claims 4 to 8, wherein said radiofrequency energy is applied at at least two different energy levels.

10. The ablation device of one of Claims 2 to 9, wherein said positioning elements are wire loops.

11. The ablation device of Claim 10, wherein said wire loops are located symmetrically about said tube.

12. The ablation device of one of Claims 3 to 11, wherein said pressing elements are wire loops.

13. The ablation device of Claim 12, wherein said wire loops are located symmetrically about said tube.

14. The ablation device of one of Claims 7 to 13, wherein said electrode members when deployed have a stem portion extending generally radially from a respective port in said tube, and a curved portion extending from said stem in an arc about said tube.

15. A method for performing ablation of a nerve at an artery ostium, as for denervation, comprising:
providing a catheter delivery mechanism including an elongated tube with a distal end and a proximal end, said distal end being emplaceable within a body lumen at a target nerve region, and having a guide wire within said elongated tube;
inserting said catheter delivery mechanism within an arterial system with the distal end at said renal artery ostium using said guide wire;
providing at least one radiofrequency electrode initially located within said tube, said electrode when deployed forming a ring-shaped structure generally centered about said tube adjacent said distal tube end;
deploying said electrode at said renal artery ostium; and
applying radiofrequency energy through said deployed electrode from said tube at said renal artery ostium in an amount to ablate tissue around said renal artery ostium.

16. The ablation method of Claim 15, further including deploying one or more positioning elements initially located within said tube from a position of said tube further distal than said electrode to position said electrode.

17. The ablation method of Claim 15, further including deploying one or more pressing elements initially located within said tube from a position more proximal than said electrode for use in pressing said deployed electrode against tissue as said target nerve region.

18. The ablation method of Claim 15, wherein said electrode is a hollow tube.

19. The ablation method of Claim 18, further including a source of coolant, and circulating said coolant through said electrode tube during ablation.

20. The ablation method of Claim 15, wherein said electrode is comprised of a plurality of separate electrode members each of which is deployable from said tube, and together take a ring-like shape when deployed.

21. The ablation method of Claim 20, wherein said electrode members are in the form of hollow tubes, further including a source of coolant, and circulating said coolant through said electrode tube member during ablation.

22. The ablation method of Claim 15, wherein said radiofrequency energy is applied at a first energy level and at least a second energy level which is different from said first energy level.

23. The ablation method of Claim 22, wherein said first and second energy levels are alternated and pulsed.

24. A method for performing ablation of a renal nerve at the renal artery ostium, comprising:
providing a catheter delivery mechanism including an elongated tube with a distal end and a proximal end, said distal end being emplaceable within the body lumen at the renal artery ostium, and having a guide wire within said elongated tube for positioning said catheter delivery mechanism;
inserting said catheter delivery mechanism with its distal end at the renal ostium;
providing at least one radiofrequency electrode initially located within said tube, said electrode when deployed forming a ring-shaped structure generally centered about said tube adjacent said distal tube end;
providing a plurality of positioning elements initially located within said tube, said positioning elements being deployable from said tube in the renal artery at the ostium from a position of said tube further distal than said electrode;
deploying said positioning elements to position said electrode;
deploying said electrode;
providing a plurality of pressing elements initially located within said tube, said pressing elements being deployable from said tube more proximal than said electrode for use in pressing said deployed electrode against ostium tissue to be ablated;
pressing said electrode against the ostium tissue;
applying radiofrequency energy through said deployed electrode from said tube in an amount to ablate the ostium tissue.

25. The ablation method of Claim 24, wherein the method is used to treat hypertension.

26. The ablation method of Claim 25, wherein said electrode is a hollow tube.

27. The ablation method of Claim 25, further including a source of coolant, and circulating said coolant through said electrode tube during ablation.

28. The ablation method of Claim 25, wherein said electrode is comprised of a plurality of separate electrode members each of which is deployable from said tube, and together take a ring-like shape when deployed.

29. The ablation method of Claim 28, wherein said electrode members are in the form of hollow tubes, further including a source of coolant, and circulating said coolant through said electrode tube member during ablation.

30. The ablation method of Claim 29, wherein said radiofrequency energy is applied at a first energy level and at least a second energy level which is different from said first energy level.
